# EUROPÄISCHE PATENTSCHRIFT

(11) **EP 2 708 248 B1**
(45) Veröffentlichungstag und Bekanntmachung des Hinweises auf die Patenterteilung: **16.11.2016**
(21) Anmeldenummer: 13004393.8
(22) Anmeldetag: 10.09.2013
(51) Int. Cl.: A61M 1/06

(54) **Manuelle Muttermilchpumpe**
Manual pump for breast milk
Pompe à lait maternel manuelle

(30) Priorität: 14.09.2012 DE 202012008803 U
(43) Veröffentlichungstag der Anmeldung: 19.03.2014
(73) Patentinhaber: MAPA GmbH, 27404 Zeven (DE)
(72) Erfinder: Jäger-Waldau, Reinhold, 27383 Scheessel (DE); Behrens, Siska, 27383 Scheessel28215 Bremen (DE)
(74) Vertreter: Hauck Patentanwaltspartnerschaft mbB

(56) Entgegenhaltungen:
- EP-A1- 1 515 760
- EP-A1- 1 818 067
- WO-A1-2009/063338
- US-A1- 2007 078 383
- US-B1- 8 187 219

## Beschreibung

Die Erfindung bezieht sich auf eine manuelle Muttermilchpumpe.

Muttermilchpumpen dienen dem Abpumpen von Muttermilch. Hierzu weisen sie mindestens eine Saugglocke auf, die auf die Mutterbrust aufgesetzt wird. An die Saugglocke wird ein Unterdruck angelegt, um die Milch aus der Brust abzuziehen. Die Saugglocke ist mit einem Milchauffangbehälter verbunden, der die abgesaugte Muttermilch aufnimmt. Der Unterdruck wird wiederholt an die Saugglocke angelegt und abgebaut, um den Milchfluss zu stimulieren. Der Unterdruck wird mittels einer Pumpe erzeugt, die bei einer manuellen Muttermilchpumpe manuell angetrieben ist. Bei einer elektrischen Muttermilchpumpe ist die Pumpe mittels eines Elektromotors angetrieben.

Bekannt sind z.B. aus der EP 1 231 955 B1 manuelle Muttermilchpumpen, bei denen die Pumpe einen Zylinder mit einem darin verlagerbaren Kolben aufweist, der mittels eines Hebels antreibbar ist. Nachteilig bei derartigen Pumpen ist die aufwendige Konstruktion und der hohe Reinigungsaufwand. Das Pumpen ist ermüdend, insbesondere weil der Reibungswiderstand zwischen Kolben und Zylinder zu überwinden ist.

Ferner sind z.B. aus der EP 1 515 760 B1 Muttermilchpumpen bekannt, bei denen die Saugglocke mit einer Verdrängungskammer verbunden ist, die abdichtend durch eine Membran verschlossen ist. Die Membran ist mittels eines Hebels auslenkbar, um in der Verdrängungskammer einen Unterdruck zu erzeugen. Durch Rückstellen der Membran in die Ausgangslage wird der Unterdruck abgebaut. Die Saugglocke ist über ein Auslassventil mit einem Milchsammelbehälter verbunden. Das Auslassventil schließt, wenn ein Unterdruck in der Verdrängungskammer vorliegt, sodass sich die Milch vor dem Ventil ansammelt. Wenn der Unterdruck abgebaut wird, öffnet das Auslassventil und die Milch läuft in den Milchsammelbehälter ab. Durch das Auslassventil wird Pumparbeit eingespart, da sich der Unterdruck nur in der Saugglocke und in der Verdrängerkammer und nicht im Milchsammelbehälter einstellt. Das Zerlegen der Muttermilchpumpe zu Reinigungszwecken und das anschließende Zusammenbauen der Pumpe ist aufwendig.

Aus EP 1 818 067 A1 oder US 8,187,219 B1 ist eine manuelle Muttermilchpumpe bekannt geworden, bei der ein becherförmiges Membranelement aus elastomerem Material vorgesehen ist, das am oberen Rand erste Mittel zum lösbaren und abdichtenden Verbinden aufweist, die mit einem oberen Rand einer Verdrängungskammer lösbar und abdichtend verbunden sind. Ein Kopplungselement ist mit dem Boden des Membranelements verbunden und das obere Ende ist mit einem ersten Hebelarm eines Hebels zum Betätigen der Pumpe verbunden.

Aus WO 2009/063338 A1 ist eine manuelle Muttermilchpumpe bekannt geworden, die ebenfalls eine becherförmige Membran aufweist, die durch eine Koppelstange betätigt wird. In der Abdeckung ist ein Einführschlitz vorgesehen, der von der Außenseite bis zur Innenseite eines Hebelarms reicht und sich vom freien Ende des Hebelarms bis auf einen Abstand von den Mitteln zum schwenkbaren Lagern des Hebelarms erstreckt, wobei die Koppelstange den Einführschlitz durchgreift und sich der Kopf des Koppelelements oberhalb des Einführschlitzes auf der Außenseite der Abdeckung abstützt. Eine derartige Ausbildung erleichtert die Demontage und das Wiederzusammensetzen der Muttermilchpumpe zwecks Reinigung. Nachteilig ist, dass die Koppelstange ein zylindrisches Lagerelement aufweist, das in der Abdeckung gelagert ist und das mit einer nach oben hin stehenden Handhabe verbunden ist. Dadurch steht ein Teil über die Abdeckung über. Außerdem erfordert die Lagerung der Koppelstange ein gezieltes Einsetzen in die Lagerausnehmung, wobei ein Verdrehen des Koppelelements mit der damit verbundenen Membran eine erhebliche Reibung verursacht.

Davon ausgehend liegt der Erfindung die Aufgabe zugrunde, eine manuelle Muttermilchpumpe zu schaffen, die insbesondere zum Zwecke der Reinigung mit verringertem Aufwand zerlegt und zusammengebaut werden kann.

Die Aufgabe wird durch eine manuelle Muttermilchpumpe mit den Merkmalen von Anspruch 1 gelöst. Vorteilhafte Ausgestaltungen der Muttermilchpumpe sind in Unteransprüchen angegeben.

Die erfindungsgemäße manuelle Muttermilchpumpe umfasst wie im Stand der Technik
- ein Gehäuse, das
- eine becherförmige Verdrängungskammer,
- einen Verbindungskanal, der oben eine Einlassöffnung und unten eine Auslassöffnung hat und zwischen Einlassöffnung und Auslassöffnung kommunizierend mit einem unteren Teil der Verdrängungskammer verbunden ist, und
- Mittel zum lösbaren Verbinden des Gehäuses mit einem Öffnungsrand eines Milchsammelbehälters unter Ausrichtung der Auslassöffnung auf die Öffnung des Milchsammelbehälters aufweist,
- einen mit den Mitteln zum lösbaren Verbinden verbundenen Milchsammelbehälter,

aufsetzt. Die Stufe dämpft das Aufschlagen des Hebels beim Erreichen der Ruhestellung.

Gemäß einer weiteren Ausgestaltung hat die Koppelstange am unteren Ende zwei Scheiben mit einem umlaufenden Ringspalt dazwischen und ist das becherförmige Membranelement am Rand eines Loches in seinem Boden zwischen den Scheiben gehalten. Diese Ausgestaltung begünstigt ein einfaches Trennen des Kopplungselements vom Membranelement und Verbinden des Kopplungselements mit dem Membranelement, beispielsweise zu Zwecken der Reinigung und des Austauschs. Alternativ ist das Membranelement dauerhaft mit dem Kopplungselement verbunden. Die Erfindung bezieht mögliche Ausgestaltungen ein, bei denen das Kopplungselement einteilig mit dem Membranelement verbunden ist. Insbesondere bezieht die Erfindung mögliche Ausgestaltungen ein, bei denen das Kopplungselement aus demselben Material wie das Membranelement besteht.

Der Kopf des Kopplungselements ist seitlich zumindest an den zu dem freien Ende des zweiten Hebelarms gerichteten Seiten mit einem Vorsprung an der Außenseite der Abdeckung abgestützt. Hierdurch wird der Kopf an einem unbeabsichtigten Herausgleiten aus dem Einführschlitz gehindert. In entgegengesetzter Richtung ist das Kopplungselement bevorzugt durch Anlage der Kopplungsstange am inneren Ende des Einführschlitzes abgestützt. Koppelelements oberhalb des Einführschlitzes auf der Außenseite der Abdeckung abstützt.

Bei der erfindungsgemäßen Lösung ist die Abdeckung einer derartigen manuellen Muttermilchpumpe kugelabschnittsförmig und der Einführschlitz erstreckt sich bis zum Scheitel der kugelabschnittsförmigen Abdeckung. Der Kopf hat die Form einer Kugelkappe und die Verbindung des Kopfes mit der Koppelstange ist über Aussteifungsrippen versteift, die radial von der Koppelstange vorstehen und mit der Unterseite des Kopfes verbunden sind. Am Ende des Einführschlitzes ist an der Außenseite der Abdeckung eine kugelschalenförmige Vertiefung vorgesehen ist, an der die Aussteifungsrippen des kugelkappenförmigen Kopfes anliegen, ferner die Außenseite der Abdeckung eine bezüglich der Vertiefung konzentrische Vertiefung aufweist, die den kugelkappenförmigen Kopf des Kopplungselements im Wesentlichen aufnimmt. Die Abdeckung weist auf der dem Einführschlitz abgewandten Seite eine Mulde neben der Vertiefung auf zum Einsetzen einer Fingerkuppe unter den Kopf des Kopplungselements.

Durch die Ausbildung des kugelkappenförmigen Kopfes, der in einer Vertiefung der kugelkappenförmigen Abdeckung liegt, werden vorstehende Teile an der Außenseite des Gehäuses vermieden. Gleichwohl lässt sich durch Einsetzen einer Fingerkuppe in eine Mulde neben der Vertiefung ein leichtes Anheben und Verschwenken der Kopplungsstange zum Auseinanderbau der Pumpe leicht bewerkstelligen. Beim Zusammenbau kann die Membran zusammen mit dem Kopplungselement in beliebiger Drehlage eingesetzt werden.

Bei der erfindungsgemäßen manuellen Brustpumpe ist das Kopplungselement mit der Koppelstange in den Einführschlitz eingesetzt und stützt sich mit dem Kopf an der Außenseite der Abdeckung ab. Dies ermöglicht ein besonders einfaches Lösen des Kopplungselements von dem Hebel durch Schwenken der Koppelstange durch den Einführschlitz hindurch und über das freie Ende des zweiten Hebelarms hinweg. Hierbei braucht die Verbindung zwischen Membranelement und Verdrängungskammer nicht gelöst zu werden, weil die Elastizität des Membranelements ein Schwenken des Kopplungselements zulässt. Umgekehrt ist das Kopplungselement mit der Koppelstange leicht vom freien Ende des zweiten Hebelarms aus in den Einführschlitz einführbar. Zudem ist das Membranelement über die ersten Mittel zum lösbaren und abdichtenden Verbinden an seinem oberen Rand mit dem oberen Rand der Verdrängungskammer lösbar und abdichtend verbunden. Nach dem Herausschwenken des Kopplungselements aus dem Einführschlitz ist diese Verbindung zwischen Membranelement und Verdrängungskammer leicht lösbar. Nach dem Lösen der Verbindung ist das Membranelement aus der Verdrängungskammer entnehmbar. Hierbei ist von Vorteil, dass der Hebel nach dem Herausschwenken des Kopplungselements aus dem Einführschlitz mit der Abdeckung von der Verdrängungskammer wegschwenkbar ist, sodass die Verdrängungskammer gut für das Entnehmen des Membranelements mit dem damit verbundenen Kopplungselement von außen zugänglich ist. Umgekehrt ist das Membranelement mit dem damit verbundenen Kopplungselement einfach in die Verdrängungskammer einsetzbar und an seinem oberen Rand lösbar und abdichtend mit dem oberen Rand der Verdrängungskammer verbindbar, wenn der Hebel mit der Abdeckung von der Verdrängungskammer weggeschwenkt ist. Die Muttermilchpumpe ist somit zu Zwecken der Reinigung oder zu Zwecken des Austausches des Membranelements leicht in Einzelteile zerlegbar. Die Teile der Muttermilchpumpe können sodann einer besonders gründlichen Reinigung unterzogen werden. Die Muttermilchpumpe hat aufgrund des Membranelements den Vorteil, dass sie ein Pumpen mit verhältnismäßig geringem Krafteinsatz ermöglicht. Beim Auswölben wird das Membranelement elastisch gedehnt. Infolgedessen kehrt das Membranelement nach Entlastung des Hebels selbsttätig in seine Ausgangsform zurück und schwenkt hierbei den Hebel in die Ruhestellung.

Der die Verdrängungskammer enthaltender Gehäuseteil ist kugelabschnittsförmig und die Abdeckung ebenfalls kugelabschnittsförmig, und der kugelabschnittsförmige Gehäuseteil und die kugelabschnittsförmige Abdeckung im Ruhezustand des Hebels im Wesentlichen die Form einer Hohlkugel aufweist. Die Kugelform hat eine angenehme Haptik. Zudem ist der Freiraum zwischen Abdeckung und Verdrängungskammer besonders groß, wenn der erste Hebelarm zum Gehäuse hin geschwenkt ist, wodurch ein Entnehmen und Einsetzen des Membranelements erleichtert wird.

Der Kopf des Kopplungselements ist in eine Vertiefung an der Außenseite der Abdeckung eingesetzt. Der Rand der Vertiefung bildet den Vorsprung, der den Kopf in Richtung auf das freie Ende des zweiten Hebelarms abstützt.

Der Kopf hat außen die Form einer Kugelkappe und weist gemeinsam mit der kugelabschnittsförmigen Abdeckung und dem kugelabschnittsförmigen Gehäuseteil im Ruhezustand des Hebels im Wesentlichen die Form einer Kugel auf. Der Kopf ergänzt die vom Gehäuseteil und der Abdeckung gebildete Kugel.

Im Abstützbereich des Kopfes ist eine Mulde vorgesehen, in die eine Fingerkuppe einsetzbar ist, um den Kopf aus der Vertiefung herauszuheben. Dies erleichtert die Demontage der Muttermilchpumpe.

Gemäß einer Ausgestaltung hat die Muttermilchpumpe ein über zweite Mittel zum lösbaren und abdichtenden Verbinden abdichtend an der Auslassöffnung gehaltenes Auslassventil, das schließt, wenn die Differenz des Druckes im Milchauffangbehälter und im Verbindungskanal einen bestimmten Mindestwert aufweist und das öffnet, wenn der Mindestwert unterschritten ist. Durch das Auslassventil wird der zum Pumpen erforderliche Krafteinsatz weiter verringert, weil im Milchauffangbehälter kein Unterdruck erzeugt wird.

Gemäß einer Ausgestaltung der Erfindung ist die Saugglocke einteilig mit dem Gehäuse verbunden, sodass Saugglocke und Gehäuse eine stabile Einheit bilden. Zu Reinigungszwecken ist es nicht erforderlich, die Saugglocke von dem Gehäuse zu trennen.

Gemäß einer weiteren Ausgestaltung ist die Saugglocke zwischen ihrer großen Öffnung und ihrer kleinen Öffnung nach außen gewölbt. Diese Konturierung der Saugglocke ist vorteilhaft für die Anpassung an die Brust. Gemäß einer weiteren Ausgestaltung ist in die Saugglocke ein trichterförmiger Einsatz aus weichelastischem Material eingesetzt, der an seiner großen Trichteröffnung über dritte Mittel zum lösbaren und abdichtenden Verbinden mit dem Rand der großen Öffnung der Saugglocke verbunden ist. Der Einsatz aus weichelastischem Material passt sich an die Form der Brust an und polstert die Saugglocke aus. Zwischen Saugglocke und Einsatz bildet sich ein Luftpolster, wenn die Saugglocke zwischen ihrer großen und ihrer kleinen Öffnung nach außen gewölbt ist, sodass der Einsatz besonders weich an der Brust anliegt.

Gemäß einer weiteren Ausgestaltung weist der trichterförmige Einsatz seine kleine Trichteröffnung in einem Hals auf, der mit seinem Außenumfang abdichtend am Innenumfang der Einlassöffnung anliegt. Infolgedessen kann beim Anlegen des Vakuums Luftpolster zwischen Einsatz und Saugglocke nicht entweichen, sodass der Einsatz besonders weich an der Brust anliegt.

Gemäß einer weiteren Ausgestaltung weist der Verbindungskanal die Einlassöffnung an einem schräg nach oben geneigten oberen Kanalabschnitt und die Auslassöffnung an einem vertikal nach unten gerichteten unteren Kanalabschnitt auf. Diese Ausgestaltung des Verbindungskanals ist vorteilhaft für die Anwendung der Muttermilchpumpe in sitzender Stellung. Die Saugglocke kann leicht nach oben geneigt an die Brust angesetzt werden, die Milch läuft durch den oberen Kanalabschnitt ab und sammelt sich im unteren Kanalabschnitt über dem Auslassventil, durch das sie in den Milchsammelbehälter abläuft. Gemäß einer bevorzugten Ausgestaltung ist der obere Kanalabschnitt in einem oberen Rohrstutzen und der untere Kanalabschnitt in einem unteren Rohrstutzen des Gehäuses ausgebildet.

Gemäß einer weiteren Ausgestaltung ist die Mittelachse der becherförmigen Verdrängungskammer auf der von der Saugglocke abgewandten Seite spitzwinklig zur Vertikalen ausgerichtet. Weiterhin vorzugsweise steht die Mittelachse der becherförmigen Verdrängungskammer senkrecht auf der Längsachse des oberen Kanalabschnitts. Dies ist vorteilhaft für eine raumsparende Anordnung der Verdrängungskammer und ein großes Verdrängungsvolumen der Membran.

Gemäß einer weiteren Ausgestaltung weisen die ersten Mittel zum lösbaren und abdichtenden Verbinden eine umlaufende Umschlagfalte am oberen Rand des Membranelements auf, in die eine umlaufende Schulter am oberen Rand der Verdrängungskammer eingreift, wobei die Umschlagfalte und die Schulter einander hintergreifende, umlaufende Hakenprofile aufweisen. Diese Ausgestaltung ist vorteilhaft für eine sichere Fixierung des Membranelements an der Verdrängungskammer, die sich beim Auswölben des Membranelements nach oben nicht löst. Dennoch ist die lösbare Verbindung zum Zerlegen der Muttermilchpumpe leicht gezielt auflösbar, indem die Umschlagfalte an einer Stelle angehoben und der obere Rand des Membranelements von der Schulter der Verdrängungskammer abgezogen wird. Umgekehrt ist das Membranelement leicht durch Ansetzen der Umschlagfalte an die Schulter und Aufdrücken auf die Schulter, bis die Hakenprofile einander hintergreifen, an der Verdrängungskammer montierbar.

Gemäß einer weiteren Ausgestaltung weist das Membranelement eine von seinem oberen Rand nach oben vorstehende, umlaufende Lippe auf, auf der die Abdeckung in Ruhestellung des Hebels aufsitzt. Die Lippe dämpft den Aufschlag des Hebels beim Erreichen der Ruhestellung. Ferner dichtet sie die Abdeckung in Ruhestellung am unteren Rand ab und verhindert die Ablagerung von Verunreinigungen auf der Oberseite des Membranelements. Zudem hält die Lippe die Abdeckung in einer vorgegebenen Ausrichtung fest und schützt damit die Mittel zum Lagern des Hebels, beispielsweise wenn die Pumpe umfällt.

Gemäß einer weiteren Ausgestaltung weist das Membranelement am oberen Rand eine umlaufende Stufe auf, auf der die Abdeckung in Ruhestellung des Hebels aufsetzt. Die Stufe dämpft das Aufschlagen des Hebels beim Erreichen der Ruhestellung.

Gemäß einer weiteren Ausgestaltung ist ein die Verdrängungskammer enthaltender Gehäuseteil kugelabschnittsförmig und die Abdeckung ebenfalls kugelabschnittsförmig und weist der kugelabschnittsförmige Gehäuseteil und die kugelabschnittsförmige Abdeckung im Ruhezustand des Hebels im Wesentlichen die Form einer Hohlkugel auf. Die Kugelform hat eine angenehme Haptik. Zudem ist der Freiraum zwischen Abdeckung und Verdrängungskammer besonders groß, wenn der erste Hebelarm zum Gehäuse hin geschwenkt ist, wodurch ein Entnehmen und Einsetzen des Membranelements erleichtert wird.

Gemäß einer weiteren Ausgestaltung hat die Koppelstange am unteren Ende zwei Scheiben mit einem umlaufenden Ringspalt dazwischen und ist das becherförmige Membranelement am Rand eines Loches in seinem Boden zwischen den Scheiben gehalten. Diese Ausgestaltung begünstigt ein einfaches Trennen des Kopplungselements vom Membranelement und Verbinden des Kopplungselements mit dem Membranelement, beispielsweise zu Zwecken der Reinigung und des Austauschs. Alternativ ist das Membranelement dauerhaft mit dem Kopplungselement verbunden. Die Erfindung bezieht mögliche Ausgestaltungen ein, bei denen das Kopplungselement einteilig mit dem Membranelement verbunden ist. Insbesondere bezieht die Erfindung mögliche Ausgestaltungen ein, bei denen das Kopplungselement aus demselben Material wie das Membranelement besteht.

Gemäß einer Ausgestaltung ist der Kopf des Kopplungselements seitlich zumindest an den zu dem freien Ende des zweiten Hebelarms gerichteten Seiten mit einem Vorsprung an der Außenseite der Abdeckung abgestützt. Hierdurch wird der Kopf an einem unbeabsichtigten Herausgleiten aus dem Einführschlitz gehindert. In Gemäß einer weiteren Ausgestaltung ist das Auslassventil ein Entenschnabelventil. Das Entenschnabelventil ist einfach, funktionssicher und leicht zu reinigen. Im Hinblick auf eine leichte Reinigung ist das Abflussventil bevorzugt am unteren Ende eines die Auslassöffnung aufweisenden unteren Rohrstutzens gehalten. Das Auslassventil ist vorzugsweise durch eine Klemmverbindung am unteren Rohrstutzen gehalten. Wenn das Auslassventil ein Entenschnabelventil ist, weist es vorzugsweise einen topfförmigen Halter auf, unter leichter elastischer Verformung auf den unteren Rohrstutzen aufgeschoben oder in diesen eingeschoben ist.

Gemäß einer weiteren Ausgestaltung besteht das becherförmige Membranelement und/oder der weichelastische Einsatz und/oder das Entenschnabelventil aus einem Silikonelastomer. Der Hebel und das Gehäuse sowie die Saugglocke bestehen gemäß einer bevorzugten Ausgestaltung aus einem im Wesentlichen starren Kunststoff.

Die Erfindung wird nachfolgend anhand der anliegenden Zeichnungen eines Ausführungsbeispiels näher erläutert. In den Zeichnungen zeigen:
- Fig. 1: eine manuelle Muttermilchpumpe im Ruhezustand des Hebels in einer Perspektivansicht schräg von vorn und von der Seite,
- Fig. 2: die Muttermilchpumpe im Ruhezustand des Hebels vertikal geschnitten in derselben Perspektivansicht;
- Fig. 3: die Muttermilchpumpe im Ruhezustand des Hebels in einem Vertikalschnitt;
- Fig. 4: die Muttermilchpumpe bei betätigtem Hebel in einer Perspektivansicht schräg von vorn und von der Seite;
- Fig. 5: die Muttermilchpumpe bei betätigtem Hebel in einem Vertikalschnitt in derselben Perspektivansicht;
- Fig. 6: die Muttermilchpumpe bei betätigtem Hebel in einem Vertikalschnitt;
- Fig. 7: die Muttermilchpumpe beim Hindurchführen der Kopplungsstange durch den Einführschlitz in einer Perspektivansicht schräg von vorn und von der Seite;
- Fig. 8: die Muttermilchpumpe beim Hindurchführen der Kopplungsstange durch den Einführschlitz im Vertikalschnitt.

In der vorliegenden Anmeldung beziehen sich die Angaben "oben" und "unten" auf eine Anordnung, bei der die Muttermilchpumpe mit einer Standfläche des Milchsammelbehälters, die an dem vom Öffnungsrand entfernten Ende des Milchsammelbehälters angeordnet ist, auf einem horizontalen Untergrund steht.

Die Muttermilchpumpe 1 weist ein Gehäuse 2 auf, das eine becherförmige Verdrängungskammer 3 aufweist. Die Verdrängungskammer 3 hat außen und im unteren Teil 4 innen eine im Wesentlichen kugelabschnittsförmige Form. Im Beispiel ist es die Form einer Halbkugel. In einem oberen Teil ist die Verdrängungskammer 3 innen zylindrisch. Am oberen Rand 5 hat die Verdrängungskammer 3 eine vorstehende umlaufende Schulter 6, die außen ein umlaufendes Hakenprofil 7 aufweist.

Ferner weist das Gehäuse 2 einen Verbindungskanal 8 auf. Der Verbindungskanal 8 hat einen oberen Kanalabschnitt 9, der in einem spitzwinklig nach oben geneigten oberen Rohrstutzen 10 angeordnet ist. Unten hat der Verbindungskanal 8 einen unteren Kanalabschnitt 11, der in einem vertikalen unteren Rohrstutzen 12 angeordnet ist. Der obere Rohrstutzen 10 und der untere Rohrstutzen 12 sind miteinander verbunden.

Die Verdrängungskammer 3 ist mit ihrer Mittelachse senkrecht zur Längsachse des oberen Rohrstutzens 10 ausgerichtet. Mit ihrem Kammerboden 13 und ihrem unteren Teil 4, in dem sie innen und außen kugelförmig ist, greift sie in den Oberbereich des oberen Rohrstutzens 10 ein.

Vom Kammerboden 13 der Verdrängungskammer 3 steht nach unten eine Spritzwand 14 in den unteren Rohrstutzen 12 hinein und unterteilt den unteren Rohrstutzen 12 im Oberbereich in der Mitte in zwei getrennte Kammern. Neben der Spritzwand 14 hat die Verdrängungskammer 3 ein Durchgangsloch 15 zum unteren Rohrstutzen 12.

Der obere Rohrstutzen 10 hat oben eine Einlassöffnung 16 des Verbindungskanals 8 und der untere Rohrstutzen 12 hat unten eine Auslassöffnung 17 des Verbindungskanals 8.

Der obere Rohrstutzen 10 ist an der Einlassöffnung 16 einteilig mit einer kleinen Öffnung einer Saugglocke 18 verbunden. Die Saugglocke 18 ist zwischen ihrer großen Öffnung 19 und ihrer kleinen Öffnung umlaufend nach außen ausgewölbt.

Das Gehäuse 2 hat unterhalb der Verdrängungskammer 3 einen umlaufenden Mantel 20, der sich nach unten allmählich erweitert. In seinem oberen Teil ist der Mantel 20 auf der Seite des oberen Rohrstutzens 10 zugleich der Mantel des unteren Rohrstutzens 12, d.h. in diesem Bereich fallen die Wände des Mantels 20 und des unteren Rohrstutzens 12 zusammen. Auf der gegenüberliegenden Seite des unteren Rohrstutzens 12 ist der Mantel 20 vom unteren Rohrstutzen 12 getrennt. Dieser Bereich beginnt etwa an den einander diametral gegenüberliegenden Stellen, an denen die Spritzwand 14 beidseits mit dem unteren Rohrstutzen 12 verbunden ist. In seinem unteren Teil erweitert sich der Mantel 20 umlaufend zu einer Haube 21. Ein unterer Teil des unteren Rohrstutzens 12 ragt in die Haube 21 hinein. Unten steht von der Innenseite der Haube 21 eine zylindrische Hülse 22 mit einem Innengewinde 23 zum Aufschrauben auf ein Außengewinde eines Milchsammelbehälters vor.

Auf der Seite der Verdrängungskammer 4, die diametral gegenüber dem oberen Rohrstutzen 10 angeordnet ist, weist das Gehäuse 2 einen rippenförmigen Träger 24 mit einer horizontalen Lagerachse 25 am oberen Ende auf, die senkrecht zur Längsachse des oberen Rohrstutzens 10 und der Saugglocke 18 ausgerichtet ist.

Die vorstehenden Elemente des Gehäuses 2 und die Saugglocke 18 sind zu einem einheitlichen Pumpenoberteil 26 fest miteinander verbunden. Vorzugsweise ist das Gehäuse 2 mit der Saugglocke 18 aus einem harten Kunststoff hergestellt, insbesondere durch Spritzgießen. Die Teile des Pumpenoberteils 26 können getrennt vorfabriziert und dann fest miteinander verbunden werden, beispielsweise durch Verschweißen.

Ferner umfasst die Muttermilchpumpe 1 ein becherförmiges Membranelement 27 aus einem elastomeren Material. Das Membranelement 27 hat einen Boden 28 mit einem zentralen Loch 29. Ausgehend vom Rand des Bodens 28 hat das Membranelement 27 einen hohlkugelabschnittsförmigen Membranabschnitt 30 und daran angrenzend einen hohlzylindrischen Membranabschnitt 31. Insoweit ist die äußere Form des Membranelements 27 an die innere Form der Verdrängungskammer 4 angepasst.

Am oberen Rand 31 weist das Membranelement 27 außen eine Umschlagfalte 32 auf. In der Umschlagfalte 32 weist das Membranelement 27 ein umlaufendes Hakenprofil 33 auf, das komplementär zum Hakenprofil 7 geformt ist und mit diesem verhakt ist.

Am oberen Rand 31 hat das Membranelement 27 eine vorstehende, umlaufende obere Lippe 33. Ferner hat das Membranelement 27 am oberen Rand 31 außerhalb der Lippe 33 eine umlaufende Stufe 34.

Angrenzend an das Loch 29 hat das Membranelement 27 eine umlaufende, hochstehende untere Lippe 35.

Das Membranelement 27 ist einteilig aus einem Silikonelastomer hergestellt.

Ferner umfasst die Muttermilchpumpe 1 ein Kopplungselement 36. Das Kopplungselement 36 weist eine Koppelstange 37 auf, die unten ein Verbindungselement 38 zum Verbinden mit dem Membranelement 27 aufweist. Das Verbindungselement 38 umfasst einen hohlzylindrischen Abschnitt 39, der über einen konischen Abschnitt 40 mit der Koppelstange 37 verbunden ist. Vom Umfang des hohlzylindrischen Abschnittes 39 stehen zwei voneinander beabstandete Scheiben 41, 42 vor. Der Boden 28 des Membranelements 27 ist am Rand des Lochs 29 in den Abstandsbereich zwischen den Scheiben 41, 42 gehalten, sodass die untere Lippe 35 am Mantel des hohlzylindrischen Abschnittes 39 und an den Scheiben 41, 42 anliegt.

Ferner umfasst das Kopplungselement 36 einen Kopf 43, der mit dem oberen Ende der Koppelstange 37 verbunden ist. Der Kopf 43 hat die Form einer Kugelkappe. Die Verbindung des Kopfes 43 mit der Koppelstange 37 ist über mehrere Aussteifungsrippen 44 versteift, die radial von der Koppelstange 37 vorstehen und mit der Unterseite des Kopfes 43 verbunden sind. Am unteren Rand haben die Aussteifungsrippen 44 einen übereinstimmenden Radius, sodass sie in einer Kugelschale schwenkbar sind.

Das Kopplungselement 36 ist einteilig aus einem harten Kunststoff hergestellt, vorzugsweise durch Spritzgießen.

Ferner umfasst die Muttermilchpumpe 1 einen Hebel 45 mit parallelen Lageraugen 46, 47 zwischen einem ersten Hebelarm 48 und einem zweiten Hebelarm 49. Die Lageraugen 46, 47 sind ösenförmig und auf die Lagerachse 25 des Gehäuses 2 aufgeklemmt.

Der erste Hebelarm 48 ist als Griff mit einem nach außen gekrümmten unteren Ende ausgebildet, das eine Hand an einem Abrutschen hindern soll. Der zweite Hebelarm 49 ist in einem kurzen Abstand von den Lageraugen 46, 47 zu einer Abdeckung 50 verbreitert. Im Bereich der Abdeckung 50 ist der Hebel kugelabschnittsförmig und in den übrigen Bereichen außen gewölbt. Ferner weist der Hebel 45 an den beiden Rändern von einer Seite vorstehende Seitenwände 51, 52 auf, die oben in die Ränder der Abdeckung 50 übergehen. Insgesamt ist der Übergang von der Abdeckung 50 zu den übrigen Teilen des Hebels 45 außen glatt.

Ferner weist der Hebel 45 einen Einführschlitz 53 auf, der vom freien Ende des zweiten Hebelarms 49 ausgeht. Der Einführschlitz 53 erstreckt sich bis zum Scheitel bzw. Pol der kugelabschnittsförmigen Abdeckung 50. Am Ende des Einführschlitzes 53 ist in der Außenseite der Abdeckung 50 eine kugelschalenförmige Vertiefung 54 vorhanden, an der die Aussteifungsrippen 44 mit ihrer Unterseite anliegen. Ferner weist die Außenseite der Abdeckung eine bezüglich der vorbezeichneten Vertiefung konzentrische Vertiefung 55 auf, die den Kopf 43 des Kopplungselements 36 im Wesentlichen aufnimmt. Zudem hat die Abdeckung 50 auf der vom Einführschlitz 53 abgewandten Seite eine Mulde 56, die zum Teil neben der letztgenannten Vertiefung 55 angeordnet ist und zum Teil in diese Vertiefung eingreift 55, sodass eine in die Mulde 56 eingesetzte Fingerkuppe den Kopf 43 des Kopplungselements 36 in der Vertiefung 55 untergreifen kann.

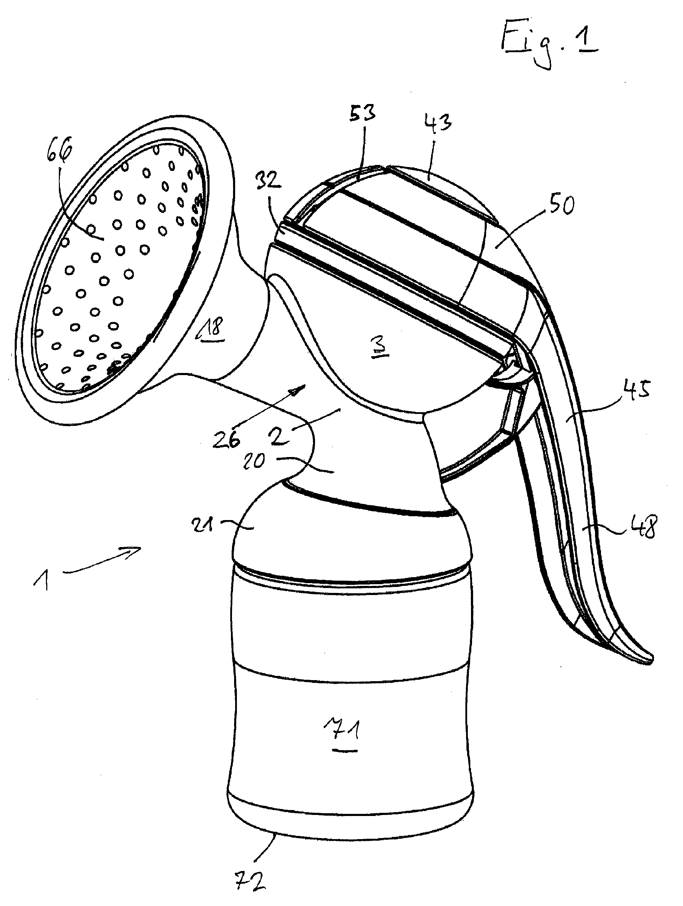

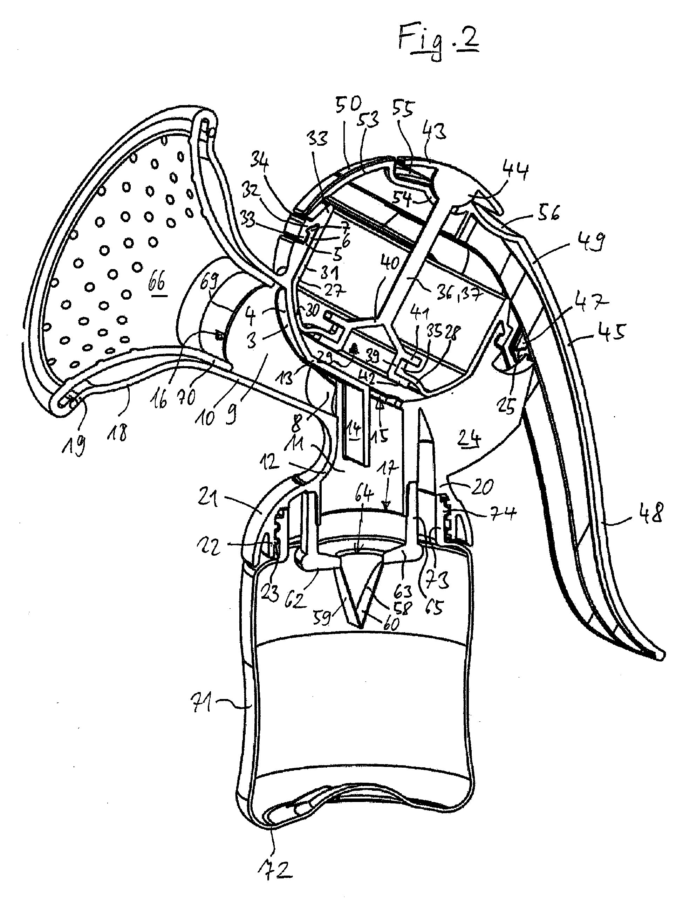

Der Hebel 45 ist mit den Lageraugen 46, 47 auf die Lagerachse 25 aufgeklemmt. Das Kopplungselement 36 hält den Hebel 45 in der Ruhestellung von Fig. 1 und 2, in der die Abdeckung 50 mit dem unteren Rand auf der Stufe 34 des Membranelements 27 aufsitzt und an der oberen Lippe 33 anliegt.

Der Hebel 45 ist aus einem harten Kunststoff hergestellt. Vorzugsweise ist er aus dem Kunststoff spritzgegossen.

Ferner umfasst die Muttermilchpumpe 1 ein Auslassventil 58, das als Entenschnabelventil ausgebildet ist. Das Entenschnabelventil ist ein Rohrstück, das zwei flache Seitenwände 59, 60 aufweist, die aufeinander zugeneigt sind und einander unten entlang einer Dichtlinie 61 berühren. Das Entenschnabelventil ist aus einem Elastomer hergestellt, vorzugsweise aus einem Silikonelastomer.

Das Auslassventil 58 steht von der Unterseite des Bodens 62 eines topfförmigen Halters 63 vor, wobei der Boden 62 im Bereich des Auslassventils 58 ein Loch 64 aufweist. Vorzugsweise ist das Auslassventil 58 einteilig mit dem Halter 63 gefertigt. Vorzugsweise bestehen Halter und Entenschnabelventil aus demselben Elastomer, insbesondere einem Silikonelastomer.

Das Auslassventil 58 ist mit einem zylindrischen Mantelteil 65 des Halters 63 abdichtend auf das untere Ende des unteren Rohrstutzens 12 aufgeklemmt.

Ferner umfasst die Muttermilchpumpe 1 einen trichterförmigen Einsatz 66 aus einem elastomeren Material. Der Einsatz 66 hat an seiner großen Öffnung 67 einen nach außen zurückgefalteten Falz 68. Mit diesem Falz 68 ist der Einsatz 66 auf den Rand der großen Öffnung 19 der Saugglocke 18 aufgeklemmt.

Der Einsatz 66 hat seine kleine Öffnung 69 in einem Hals 70, der am Außenumfang abdichtend am oberen Rohrstutzen 10 anliegt.

Zwischen dem Einsatz 66 und der Saugglocke 18 ist ein umlaufendes Luftpolster vorhanden.

Der Einsatz 66 ist vorzugsweise einteilig aus einem elastomeren Material hergestellt, insbesondere aus einem Silikonelastomer.

Schließlich umfasst die Muttermilchpumpe 1 einen Milchsammelbehälter 71. Der Milchsammelbehälter 71 hat eine Flaschenform. Unten weist er eine Standfläche 72 und oben einen Hals 73 mit einem Außengewinde 74 auf. Das Gehäuse 2 ist mit dem Innengewinde 23 auf das Außengewinde 74 aufgeschraubt. Der Milchsammelbehälter 71 kann nach dem Befüllen mit Milch auch als Saugflasche verwendet werden.

Der Milchsammelbehälter 71 ist bevorzugt aus einem starren Kunststoff hergestellt. Vorzugsweise ist er einteilig aus dem Kunststoff spritzgegossen.

Für das Abpumpen von Milch wird die Muttermilchpumpe 1 mit der Saugglocke 18 auf eine Brust aufgesetzt, sodass diese an der Außenseite des Einsatzes 66 anliegt. Dann wird zyklisch der Hebel 45 betätigt, in dem der erste Hebelarm 48 in Richtung auf das Gehäuse 7 bzw. den Milchsammelbehälter 71 gedrückt und anschließend entlastet wird.

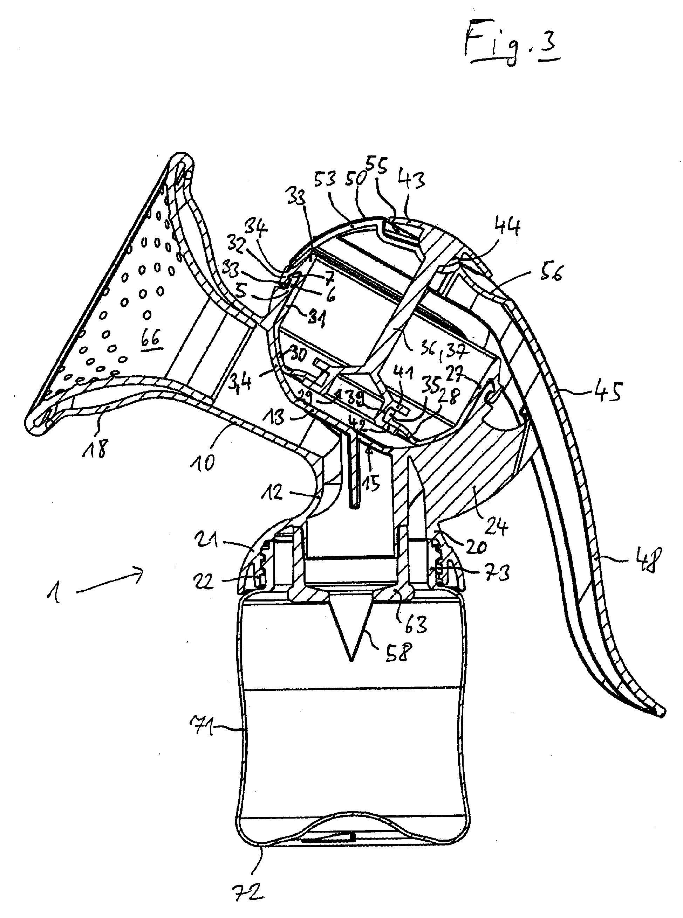

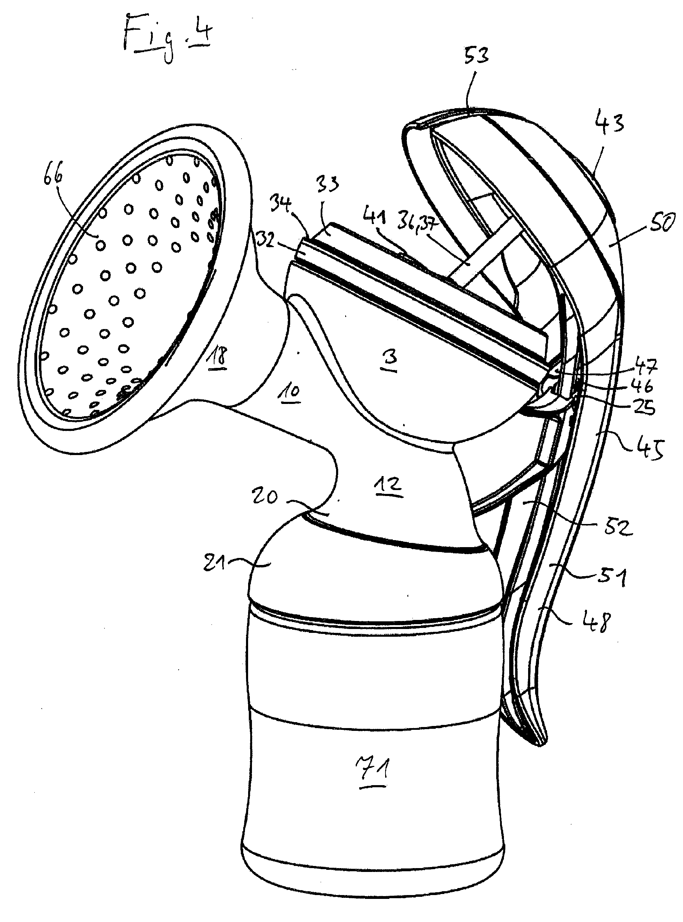

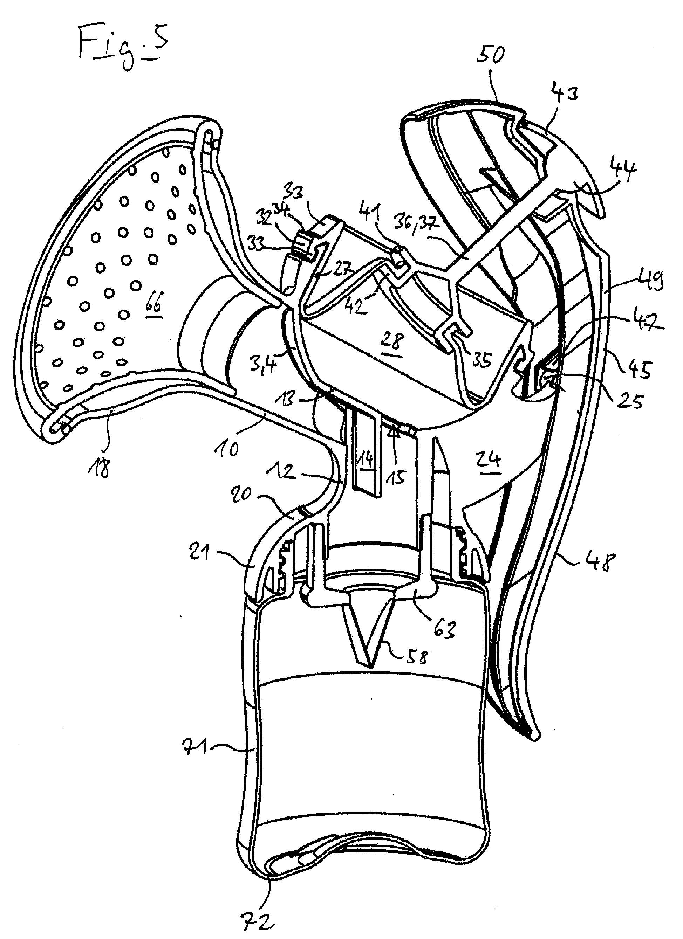

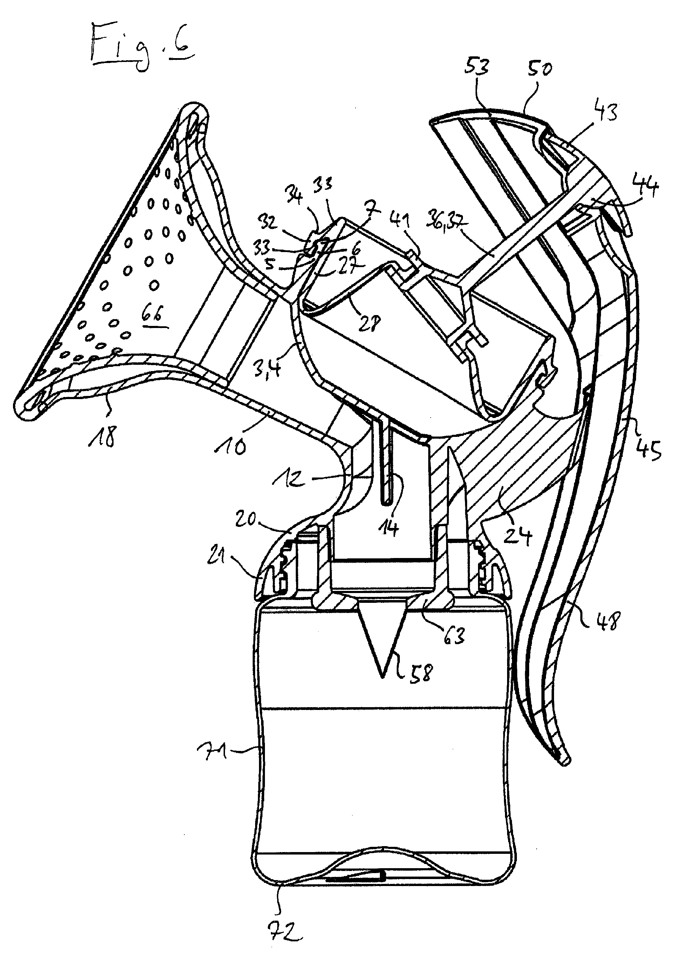

Durch Betätigen des Hebels 45 wird das Membranelement 27 nach oben ausgelenkt, so wie in den Fig. 4, 5, 6 gezeigt. Hierdurch wird in der Verdrängungskammer 3 und in der Saugglocke 18 ein Unterdruck erzeugt. Nach Entlastung des Hebels 45 nimmt das Membranelement 27 seine Ausgangsform an und zieht den Hebel 45 in die Ruhestellung von Fig. 1, 2, 3 zurück. Hierbei wird der Unterdruck in der Verdrängungskammer 3 und in der Saugglocke 18 wieder abgebaut.

Durch zyklisches Anlegen und Abbauen des Unterdruckes wird der Milchfluss stimuliert. Die Milch läuft durch den Einsatz 66 und den Verbindungskanal 8 in das Entenschnabelventil 58 ab. Wenn Unterdruck in der Verdrängungskammer 3 und damit im Verbindungskanal 8 vorliegt, schließt das Entenschnabelventil 58 und hält die Milch fest. Wenn der Unterdruck abgebaut wird, öffnet das Entenschnabelventil 58 und lässt die Milch in den Milchsammelbehälter 71 ab.

Nach dem Abpumpen der Milch wird das Gehäuse 2 vom Milchsammelbehälter 71 abgeschraubt und der Milchsammelbehälter 71 zum Zwecke der Aufbewahrung der Milch geschlossen oder mit einem Trinksauger versehen werden, um die Milch zu verabreichen.

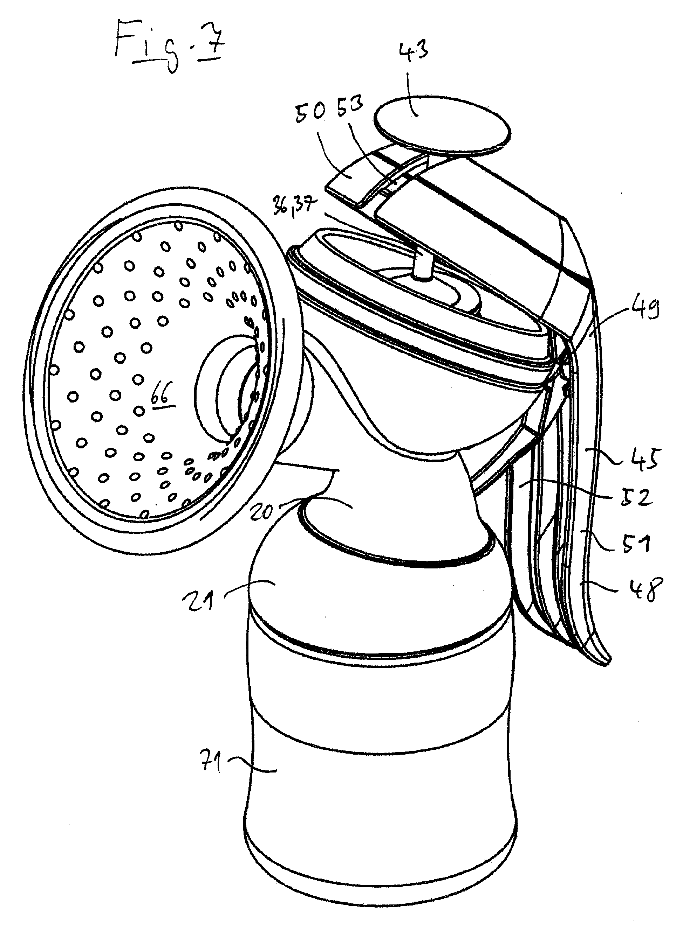

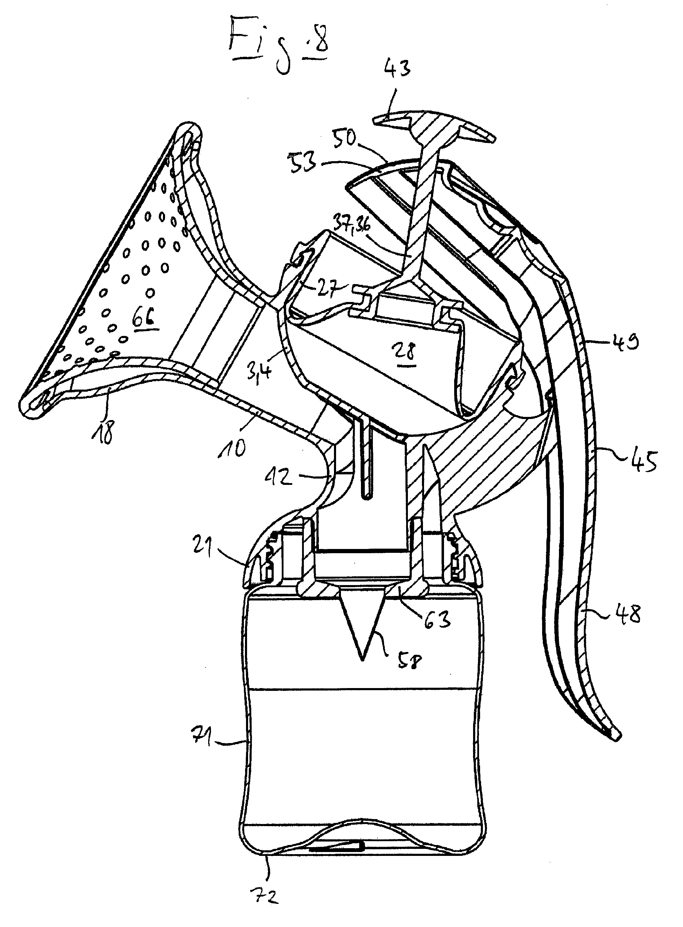

Zur Reinigung der Muttermilchpumpe 1 wird das Gehäuse 2 vom Milchsammelbehälter 71 abgeschraubt. Ferner wird das Kopplungselement 36 vom Hebel 45 gelöst, indem der Kopf 43 mit der Fingerkuppe aus dem Einführschlitz 53 herausgeschwenkt wird, wie in Fig. 7, 8 gezeigt. Anschließend ist das Membranelement 27 durch Abdrücken von der Schulter 6 vom Gehäuse 2 lösbar.

Das Entenschnabelventil 58 ist durch Abdrücken vom unteren Rohrstutzen 12 vom Gehäuse 2 trennbar.

Der Einsatz 66 ist durch Abdrücken vom oberen Rand der Saugglocke 18 ablösbar.

Der Hebel 45 kann durch Abdrücken der Lageraugen 46, 47 von der Lagerachse 25 des Gehäuses 2 getrennt werden.

Der Milchsammelbehälter 71 ist durch Abschrauben vom Gehäuse 2 lösbar.

Die vorbezeichneten Einzelteile können einzeln gereinigt und ggfs. sterilisiert werden. Danach sind sie in umgekehrter Abfolge leicht zusammenbaubar.

## Patentansprüche

1. Manuelle Muttermilchpumpe umfassend
- ein Gehäuse (2), das
- eine becherförmige Verdrängungskammer (3),
- einen Verbindungskanal (8), der oben eine Einlassöffnung (16) und unten eine Auslassöffnung (17) hat und zwischen Einlassöffnung (16) und Auslassöffnung (17) kommunizierend mit einem unteren Teil der Verdrängungskammer (3) verbunden ist und
- Mittel zum lösbaren Verbinden (23) des Gehäuses (2) mit einem Öffnungsrand (73) eines Milchsammelbehälters (71) aufweist,
- einen mit den Mitteln zum lösbaren Verbinden (23) verbundenen Milchsammelbehälter (71),
- eine Saugglocke (18), die zum Aufnehmen eines Teils einer Brust ausgestaltet ist und mit der Einlassöffnung (16) des Verbindungskanals (8) verbunden ist,
- ein becherförmiges Membranelement (27) aus einem elastomeren Material, das am oberen Rand (31) erste Mittel zum lösbaren und abdichtenden Verbinden (32, 33) aufweist, die mit einem oberen Rand der Verdrängungskammer (3) lösbar und abdichtend verbunden sind,
- ein Kopplungselement (36), das eine Koppelstange (37) aufweist, die unten mit einem Boden (28) des becherförmigen Membranelementes (27) verbunden ist, und das einen Kopf (43) aufweist, der mit dem oberen Ende (37) der Koppelstange verbunden ist und seitlich über die Koppelstange (37) hinaussteht,
- einen Hebel (45) mit einem ersten Hebelarm (48) und einem zweiten Hebelarm (49), wobei der zweite Hebelarm (49) eine auf den oberen Rand des Membranelementes (27) aufsetzbare Abdeckung (50) umfasst,
- Mittel zum schwenkbaren Lagern (25, 46, 47) des Hebels (45), die den Hebel (45) zwischen dem ersten Hebelarm (48) und dem zweiten Hebelarm (49) schwenkbar am Gehäuse (2) lagern, wobei in Ruhestellung des Hebels (49) der erste Hebelarm (48) seitlich in einem Abstand vom Gehäuse (2) angeordnet ist und der zweite Hebelarm (49) mit der Abdeckung (50) auf dem oberen Rand des Membranelements (27) angeordnet ist, so dass der Hebel (45) durch Schwenken des ersten Hebelarmes (48) zum Gehäuse (2) hin mit dem zweiten Hebelarm (49) von der Verdrängungskammer (3) weg schwenkbar ist und dabei über das Kopplungselement (36) einen Teil des Membranelements (27) innerhalb seines oberen Randes (31) nach oben auswölbt, und
- einen Einführschlitz (53), der von der Außenseite bis zur Innenseite des zweiten Hebelarms (49) reicht und sich vom freien Ende (54) des zweiten Hebelarms (49) bis auf einen Abstand von den Mitteln zum schwenkbaren Lagern (25, 46, 47) erstreckt, wobei die Koppelstange (37) den Einführschlitz (53) durchgreift und sich der Kopf (43) des Koppelelementes (36) oberhalb des Einführschlitzes (53) auf der Außenseite der Abdeckung (50) abstützt,
**dadurch gekennzeichnet,**
**dass** die Abdeckung (50) kugelabschnittsförmig ist und der Einführschlitz (53) sich bis zum Scheitel der kugelabschnittsförmigen Abdeckung (50) erstreckt, und dass der Kopf (43) die Form einer Kugelkappe hat und die Verbindung des Kopfes (43) mit der Koppelstange (37) über Aussteifungsrippen (44) versteift ist, die radial von der Koppelstange (37) vorstehen und mit der Unterseite des Kopfes (43) verbunden sind und dass am Ende des Einführschlitzes an der Außenseite der Abdeckung (50) eine kugelschalenförmige Vertiefung (54) vorgesehen ist, an der die Aussteifungsrippen des kugelkappenförmigen Kopfes (43) anliegen, und ferner die Außenseite der Abdeckung eine bezüglich der Vertiefung (54) konzentrische Vertiefung (55) aufweist, die den kugelkappenförmigen Kopf des Kopplungselements (36) im Wesentlichen aufnimmt und die Abdeckung auf der dem Einführschlitz abgewandten Seite eine Mulde neben der Vertiefung aufweist zum Einsetzen einer Fingerkuppe unter den Kopf des Kopplungselements.

2. Muttermilchpumpe nach Anspruch 1, umfassend ein über zweite Mittel zum lösbaren und abdichtenden Verbinden (63) abdichtend an der Auslassöffnung (17) gehaltenes Auslassventil (58), das schließt, wenn die Differenz des Druckes im Milchauffangbehälter (71) und im Verbindungskanal (8) einen bestimmten Mindestwert aufweist und das öffnet, wenn der Mindestwert unterschritten ist.

3. nach Anspruch 1 oder 2, bei der die Saugglocke (18) einteilig mit dem Gehäuse (2) verbunden ist.

4. Muttermilchpumpe nach einem der Ansprüche 1 bis 3, bei der die Saugglocke (18) zwischen ihrer großen Öffnung und ihrer kleinen Öffnung nach außen gewölbt ist.

5. Muttermilchpumpe nach einem der Ansprüche 1 bis 4, bei der in die Saugglocke (18) ein trichterförmiger Einsatz (66) aus weichelastischem Material eingesetzt ist, der an seiner großen Trichteröffnung (67) über dritte Mittel zum lösbaren und abdichtenden Verbinden (68) mit dem Rand der großen Öffnung der Saugglocke (18) verbunden ist.

6. Muttermilchpumpe nach Anspruch 5, bei der der trichterförmige Einsatz (66) seine kleine Öffnung (69) in einem Hals (70) aufweist, der mit seinem Außenumfang abdichtend am Innenumfang der Einlassöffnung (16) anliegt.

7. Muttermilchpumpe nach einem der Ansprüche 1 bis 6, bei der der Verbindungskanal (8) die Einlassöffnung (16) an einem schräg nach oben geneigten oberen Kanalabschnitt (9) und die Auslassöffnung an einem vertikal nach unten gerichteten unteren Kanalabschnitt (10) aufweist.

8. Muttermilchpumpe nach einem der Ansprüche 1 bis 7, bei der die Mittelachse der becherförmigen Verdrängungskammer (3) auf der von der Saugglocke (18) abgewandten Seite spitzwinklig zur Vertikalen geneigt ist.

9. Muttermilchpumpe nach einem der Ansprüche 1 bis 8, bei der die ersten Mittel zum lösbaren und abdichtenden Verbinden eine umlaufende Umschlagfalte (32) am oberen Rand des Membranelements (27) aufweisen, in die eine umlaufende Schulter (6) am oberen Rand der Verdrängungskammer (3) eingreift, wobei die Umschlagfalte (32) und die Schulter (6) einander hintergreifende, umlaufende Hakenprofile (7, 33) aufweisen.

10. Muttermilchpumpe nach einem der Ansprüche 1 bis 9, bei der das Membranelement (27) eine von seinem oberen Rand nach oben vorstehende, umlaufende obere Lippe (33) aufweist, auf der die Abdeckung (50) in Ruhestellung des Hebels (45) aufsitzt.

11. Muttermilchpumpe nach einem der Ansprüche 1 bis 10, bei der das Membranelement (27) am oberen Rand eine umlaufende Stufe (34) aufweist, auf der die Abdeckung (50) in Ruhestellung des Hebels (45) aufsitzt.

12. Muttermilchpumpe nach einem der Ansprüche 1 bis 11, bei der ein die Verdrängungskammer (3) enthaltender Gehäuseteil kugelabschnittsförmig ist und das kugelabschnittsförmige Gehäuseteil und die kugelabschnittsförmige Abdeckung (50) gemeinsam im Ruhezustand des Hebels (45) im Wesentlichen die Form einer Hohlkugel aufweisen.

13. Muttermilchpumpe nach einem der Ansprüche 1 bis 12, bei der die Koppelstange (37) am unteren Ende zwei Scheiben (41, 42) mit einem umlaufenden Ringspalt dazwischen aufweist und das becherförmige Membranelement (27) am Rand eines Loches (29) in seinem Boden (28) zwischen den Scheiben (41, 42) gehalten ist.

14. Muttermilchpumpe nach Anspruch 1, bei der der Kopf (43) außen die Form einer Kugelkappe hat und gemeinsam mit der kugelabschnittsförmigen Abdeckung (50) und dem außen kugelabschnittsförmigen Gehäuseteil im Ruhezustand des Hebels (45) im Wesentlichen die Form einer Kugel aufweist.

## Claims

1. Manual breast milk pump comprising
- a housing (2), which
- has a cup-like displacement chamber (3),
- has a connection channel (8), which has an inlet opening (16) on the top and an outlet opening (17) on the bottom, and is connected in a communicating manner with a bottom part of the displacement chamber (3) between the inlet opening (16) and the outlet opening (17) and
- has means for the releasable connection (23) of the housing (2) with an opening edge (73) of a milk collection container (71),
- a milk collection container (71) connected with the means for releasable connection (23),
- a suction bell (18), which is designed to receive a part of a breast and is connected with the inlet opening (16) of the connection channel (8),
- a cup-like membrane element (27) made of an elastomer material, which has on its upper edge (31) first means for releasable and sealing connection (32, 33), which are connected in a releasable and sealing manner with an upper edge of the displacement chamber (3),
- a coupling element (36), which has a coupling rod (37), which is connected on the bottom to a bottom (28) of the cup-like membrane element (27), and said coupling element having a head (43), which is connected with the upper end (37) of the coupling rod and protrudes laterally over the coupling rod (37),
- a lever (45) with a first lever arm (48) and second lever arm (49), wherein the second lever arm (49) comprises a cover (50) placeable on the upper edge of the membrane element (27),
- means for the rotatable mounting (25, 46, 47) of the lever (45), which mount the lever (45) rotatably between the first lever arm (48) and the second lever arm (49) on the housing (2), wherein in the lever's idle position (49) the first lever arm (48) is arranged laterally at a distance from the housing (2) and the second lever arm (49) is arranged with the cover (50) on the upper edge of the membrane element (27) so that the lever (45) through rotation of the first lever arm (48) towards the housing (2) is rotatable with the second lever arm (49) away from the displacement chamber (3) and thereby bulges a part of the membrane element (27) within its upper edge (31) upwards via the coupling element (36) and
- an insertion slot (53), which reaches from the outside up to the inside of the second lever arm (49) and extends from the free end (54) of the second lever arm (49) up to a distance from the means for rotatable mounting (25, 46, 47), wherein the coupling rod (37) reaches through the insertion slot (53) and the head (43) of the coupling element (36) above the insertion slot (53) is supported on the outside of the cover (50),
**characterized in that**
the cover (50) is shaped as a spherical segment and the insertion slot (53) extends up to the crest of the cylindrical-segment-shaped cover (50), and the head (43) is shaped as spherical calotte and the connection of the head (43) with the coupling rod (37) is stiffened through ribs (44) which protrude radially from the coupling rod (37) and are connected with the lower side of the head (43) and at the end of the insertion slot at the outer side of the cover (50) a spherical-shell-shaped recess (54) is formed which is engaged by the stiffening ribs of the head (43), and further the outer side of the cover has a recess (55) concentric with respect to the spherical-shell-shaped recess (54) which substantially accommodates the head of the coupling element (36), and the cover at a side facing away from the insertion slot has a trough, next to the recess, for inserting a fingertip into the trough below the head of the coupling element.

2. The breast milk pump according to claim 1, comprising an outlet valve (58) held sealed on the outlet opening (17) via a second means for releasable and sealing connection (63) that closes when the difference in the pressure in the milk collection container (71) and in the connection channel (8) has a certain minimum value and that opens when the minimum value is fallen short of.

3. The breast milk pump according to claim 1 or 2, in which the suction bell (18) is connected with the housing (2) as one single part.

4. The breast milk pump according to one of claims 1 through 3, in which the suction bell (18) bulges outward between its large opening and its small opening.

5. The breast milk pump according to one of claims 1 through 4, in which a funnel-shaped insert (66) made of soft elastic material is inserted into the suction bell (18), said funnel-shaped insert being connected on its large funnel opening (67) via third means for releasable and sealing connection (68) with the edge of the large opening of the suction bell (18).

6. The breast milk pump according to claim 5, in which the funnel-shaped insert (66) has its small opening (69) in a neck (70), which rests with its outer circumference in a sealing manner on the inner circumference of the inlet opening (16).

7. The breast milk pump according to one of claims 1 through 6, in which the connection channel (8) has the inlet opening (16) on a diagonally upward sloping upper channel section (9) and the outlet opening on a vertically downward pointing lower channel section (10).

8. The breast milk pump according to one of claims 1 through 7, in which the centre axis of the cup-like displacement chamber (3) is aligned at an acute angle to the vertical line on the side facing away from the suction bell (18).

9. The breast milk pump according to one of claims 1 through 8, in which the first means for releasable and sealing connection has a circumferential collar fold (32) on the upper edge of the membrane element (27), into which a circumferential shoulder (6) on the upper edge of the displacement chamber (3) engages, wherein the collar fold (32) and the shoulder (6) have circumferential hook profiles (7, 33) engaging behind each other.

10. The breast milk pump according to one of claims 1 through 9, in which the membrane element (27) has a circumferential upper lip (33) protruding upwards from its upper edge, on which the cover (50) sits in the idle position of the lever (45).

11. The breast milk pump according to one of claims 1 through 10, in which the membrane element (27) has a circumferential step (34) on the upper edge, on which the cover (50) sits in the idle position of the lever (45).

12. The breast milk pump according to one of claims 1 through 11, in which a housing part containing the displacement chamber (3) is spherical-segment-shaped and the cover (50) is also spherical-segment-shaped and the spherical-segment-shaped housing part and the spherical-segment-shaped cover (50) have together in the idle state of the lever (45) mainly the shape of a hollow sphere.

13. The breast milk pump according to one of claims 1 through 12, in which the coupling rod (37) has two discs (41, 42) with a circumferential annular gap in between on the lower end and the cup-like membrane element (27) is held on the edge of a hole (29) in its bottom (28) between the discs (41, 42).

14. The breast milk pump according to claim 1, in which the head (43) has at the outer side the shape of a spherical calotte and together with the spherical-segmented cover (50) and the externally spherical-segmented housing part in the idle position of the lever (45) has essentially the shape of a sphere.

## Revendications

1. Pompe à lait maternel manuelle comprenant
- un logement (2) qui présente
- une chambre de refoulement en forme de gobelet (3),
- un canal de connexion (8) qui a en haut une ouverture d'entrée (16) et en bas une ouverture de sortie (17), et est relié de manière communicante à une partie inférieure de la chambre de refoulement (3) entre ouverture d'entrée (16) et ouverture de sortie (17), et
- des moyens pour la connexion amovible (23) du logement (2) à un bord d'ouverture (73) d'un récipient de collecte de lait (71),
- un récipient de collecte de lait (71) relié aux moyens pour la connexion amovible (23),
- une cloche d'aspiration (18) qui est configurée pour recevoir une partie d'un sein et est reliée à l'ouverture d'entrée (16) du canal de connexion (8),
- un élément de membrane en forme de gobelet (27) en un matériau élastomère qui présente au niveau du bord supérieur (31) des premiers moyens pour la connexion amovible et étanchéifiante (32, 33) qui sont reliés de manière amovible et étanchéifiante à un bord supérieur de la chambre de refoulement (3),
- un élément d'accouplement (36) qui présente une bielle d'accouplement (37), qui est reliée en bas à un fond (28) de l'élément de membrane en forme de gobelet (27), et qui présente une tête (43) qui est reliée à l'extrémité supérieure (37) de la bielle d'accouplement et dépasse latéralement au-delà de la bielle d'accouplement (37),
- un levier (45) avec un premier bras de levier (48) et un second bras de levier (49), dans laquelle le second bras de levier (49) comprend un recouvrement (50) pouvant être posé sur le bord supérieur de l'élément de membrane (27),
- des moyens pour le logement pivotant (25, 46, 47) du levier (45) qui logent le levier (45) entre le premier bras de levier (48) et le second bras de levier (49) à pivotement sur le logement (2), dans laquelle le premier bras de levier (48) est disposé latéralement à un écart du logement (2) en position de repos du levier (49) et le second bras de levier (49) est disposé avec le recouvrement (50) sur le bord supérieur de l'élément de membrane (27) de sorte que le levier (45) est pivotant par pivotement du premier bras de levier (48) vers le logement (2) avec le second bras de levier (49) à l'écart de la chambre de refoulement (3) et bombe ce faisant par le biais de l'élément d'accouplement (36) une partie de l'élément de membrane (27) au sein de son bord supérieur (31) vers le haut, et
- une fente d'introduction (53) qui va du côté externe jusqu'au côté interne du second bras de levier (49) et s'étend de l'extrémité libre (54) du second bras de levier (49) à l'exception d'un écart des moyens pour le logement pivotant (25, 46, 47), dans laquelle la bielle d'accouplement (37) pénètre dans la fente d'introduction (53) et la tête (43) de l'élément d'accouplement (36) s'appuie au-dessus de la fente d'introduction (53) sur le côté externe du recouvrement (50),
**caractérisée en ce**
**que** le recouvrement (50) est en forme de segment sphérique et la fente d'introduction (53) s'étend jusqu'au sommet du recouvrement en forme de segment sphérique (50), et que la tête (43) a la forme d'une calotte sphérique et la connexion de la tête (43) à la bielle d'accouplement (37) est renforcée par le biais de nervures de renforcement (44) qui font saillie radialement de la bielle d'accouplement (37) et sont reliées au côté inférieur de la tête (43), et qu'un évidement en forme de cuvette (54) est prévu à l'extrémité de la fente d'introduction sur le côté externe du recouvrement (50), sur lequel les nervures de renforcement de la tête en forme de calotte sphérique (43) reposent, et le côté externe du recouvrement présente en outre un évidement (55) concentrique par rapport à l'évidement (54) qui reçoit essentiellement la tête en forme de calotte sphérique de l'élément d'accouplement (36) et le recouvrement présente sur le côté détourné de la fente d'introduction un moule à côté de l'évidement pour l'insertion d'un bout de doigt sous la tête de l'élément d'accouplement.

2. Pompe à lait maternel selon la revendication 1, comprenant une soupape de sortie (58) maintenue de manière étanchéifiante sur l'ouverture de sortie (17) par le biais de deuxièmes moyens pour la connexion amovible et étanchéifiante (63) qui se ferme lorsque la différence de la pression dans le récipient de collecte de lait (71) et dans le canal de connexion (8) présente une valeur minimale définie et qui s'ouvre lorsque la valeur minimale est dépassée par le bas.

3. Pompe à lait maternel selon la revendication 1 ou 2, dans laquelle la cloche d'aspiration (18) est reliée d'une pièce au logement (2).

4. Pompe à lait maternel selon une des revendications 1 à 3, dans laquelle la cloche d'aspiration (18) est bombée vers l'extérieur entre sa grande ouverture et sa petite ouverture.

5. Pompe à lait maternel selon une des revendications 1 à 4, dans laquelle un insert en forme d'entonnoir (66) en matériau élastique souple est inséré dans la cloche d'aspiration (18), lequel est relié au niveau de sa grande ouverture d'entonnoir (67) par le biais de troisièmes moyens pour la connexion amovible et étanchéifiante (68) au bord de la grande ouverture de la cloche d'aspiration (18).

6. Pompe à lait maternel selon la revendication 5, dans laquelle l'insert en forme d'entonnoir (66) présente sa petite ouverture (69) dans un col (70) qui repose avec sa périphérie externe de manière étanchéifiante sur la périphérie interne de l'ouverture d'entrée (16).

7. Pompe à lait maternel selon une des revendications 1 à 6, dans laquelle le canal de connexion (8) présente l'ouverture d'entrée (16) au niveau d'une section de canal supérieure (9) inclinée en biais vers le haut et l'ouverture de sortie au niveau d'une section de canal inférieure (10) orientée verticalement vers le bas.

8. Pompe à lait maternel selon une des revendications 1 à 7, dans laquelle l'axe médian de la chambre de refoulement en forme de gobelet (3) est incliné à angle aigu par rapport à la verticale sur le côté détourné de la cloche d'aspiration (18).

9. Pompe à lait maternel selon une des revendications 1 à 8, dans laquelle les premiers moyens pour la connexion amovible et étanchéifiante présentent un pli de couverture périphérique (32) au niveau du bord supérieur de l'élément de membrane (27) dans lequel un épaulement périphérique (6) s'engrène au niveau du bord supérieur de la chambre de refoulement (3), dans laquelle le pli de couverture (32) et l'épaulement (6) présentent des profils de crochet périphériques (7, 33) s'engageant les uns dans les autres.

10. Pompe à lait maternel selon une des revendications 1 à 9, dans laquelle l'élément de membrane (27) présente une lèvre périphérique supérieure (33) faisant saillie vers le haut depuis son bord supérieur sur laquelle le recouvrement (50) repose en position de repos du levier (45).

11. Pompe à lait maternel selon une des revendications 1 à 10, dans laquelle l'élément de membrane (27) présente un gradin périphérique (34) au niveau du bord supérieur sur lequel le recouvrement (50) repose en position de repos du levier (45).

12. Pompe à lait maternel selon une des revendications 1 à 11, dans laquelle une partie de logement contenant la chambre de refoulement (3) est en forme de segment sphérique, et la partie de logement en forme de segment sphérique et le recouvrement en forme de segment sphérique (50) présentent ensemble à l'état de repos du levier (45) essentiellement la forme d'une sphère creuse.

13. Pompe à lait maternel selon une des revendications 1 à 12, dans laquelle la bielle d'accouplement (37) présente à l'extrémité inférieure deux disques (41, 42) avec un espace annulaire périphérique entre eux et l'élément de membrane en forme de gobelet (27) est maintenu entre les disques (41, 42) au niveau du bord d'un orifice (29) dans son fond (28).

14. Pompe à lait maternel selon la revendication 1, dans laquelle la tête (43) a à l'extérieur la forme d'une calotte sphérique et présente essentiellement la forme d'une sphère avec le recouvrement en forme de segment sphérique (50) et la partie de logement en forme de segment sphérique à l'extérieur à l'état de repos du levier (45).
